# EUROPEAN PATENT SPECIFICATION

(11) **EP 1 295 895 B1**
(45) Date of publication and mention of the grant of the patent: **10.08.2011**
(21) Application number: 01402406.1
(22) Date of filing: 19.09.2001
(51) Int. Cl.: C07K 14/435, C12N 15/00, G01N 33/00, A61K 38/00, A61K 39/00

(54) **Peptides and proteins binding to glu-pro motifs, therapeutical compositions containing the same and applications thereof**
An das Glu-Pro Motiv-bindende Proteine und Peptide, diese enthaltende therapeutische Zusammensetzungen und deren Anwendungen
Peptides et proteines se liant aux motifs glu-pro, compositions thérapeutiques les comprenant et leurs applications

(43) Date of publication of application: 26.03.2003
(73) Proprietor: Institut Gustave Roussy, 94805 Villejuif Cédex (FR); UNIVERSITE PARIS-SUD (PARIS XI), 91405 Orsay Cédex (FR)
(72) Inventor: Triebel, Frédéric, 78000 Versailles (FR)
(74) Representative: Novagraaf Technologies

(56) References cited:
- EP-A- 0 893 507
- EP-A- 0 900 841
- WO-A-97/03695
- FUKAMACHI HIROMI ET AL: "Identification of a protein, SYP75, with repetitive helix-turn-helix motifs and an SH3 domain as a major substrate for protein tyrosine kinase(s) activated by Fc-epsilon-RI cross-linking." JOURNAL OF IMMUNOLOGY, vol. 152, no. 2, 1994, pages 642-652, XP002209777 ISSN: 0022-1767
- PEARSON T W ET AL: "PROCYCLIN: AN IMMUNODOMINANT TRYPANOSOME SURFACE ANTIGEN WITH POTENTIAL FOR USE AS A TRANSMISSION BLOCKING VACCINE" UCLA SYMPOSIA ON MOLECULAR AND CELLULAR BIOLOGY, NEW YORK, NY, US, vol. 84, 30 January 1988 (1988-01-30), pages 45-56, XP001022687 ISSN: 0735-9543
- GAISSER, S. ET AL: "The tonB gene of Serratia marcescens: sequence, activity and partial complementation of Escherichia coli tonB mutants" MOL. MICROBIOL. (1991), 5(11), 2777-87 , 1991, XP001094991
- FERRIERES G ET AL: "AFFINITY FOR THE COGNATE MONOCLONAL ANTIBODY OF SYNTHETIC PEPTIDES DERIVED FROM SELECTION BY PHAGE DISPLAY ROLE OF SEQUENCES FLANKING THE BINDING MOTIF" EUROPEAN JOURNAL OF BIOCHEMISTRY, BERLIN, DE, vol. 6, no. 267, 2000, pages 1819-1829, XP001093880 ISSN: 0014-2956
- HANNIER SIGRID ET AL: "The MHC class II ligand lymphocyte activation gene-3 is co-distributed with CD8 and CD3-TCR molecules after their engagement by mAb or peptide-MHC class I complexes." INTERNATIONAL IMMUNOLOGY, vol. 11, no. 11, November 1999 (1999-11), pages 1745-1752, XP002209778 ISSN: 0953-8178
- HUARD B ET AL: "T CELL MAJOR HISTOCOMPATIBILITY COMPLEX CLASS II MOLECULES DOWN-REGULATE CD4+ T CELL CLONE RESPONSES FOLLOWING LAG-3 BINDING" EUROPEAN JOURNAL OF IMMUNOLOGY, WEINHEIM, DE, vol. 26, 1 May 1996 (1996-05-01), pages 1180-1186, XP000672155 ISSN: 0014-2980
- MANGASARIAN ARAM ET AL: "Nef-induced CD4 and major histocompatibility complex class I (MHC-1) down-regulation are governed by distinct determinants: N-terminal alpha helix and proline repeat of Nef selectively regulate MHC-1 trafficking." JOURNAL OF VIROLOGY, vol. 73, no. 3, March 1999 (1999-03), pages 1964-1973, XP002209779 ISSN: 0022-538X
- HUARD B ET AL: "CHARACTERIZATION OF THE MAJOR HISTOCOMPATIBILITY COMPLEX CLASS II BINDING SITE ON LAG-3 PROTEIN" PROCEEDINGS OF THE NATIONAL ACADEMY OF SCIENCES OF USA, NATIONAL ACADEMY OF SCIENCE. WASHINGTON, US, vol. 94, no. 11, 1 May 1997 (1997-05-01), pages 5744-5749, XP002037067 ISSN: 0027-8424
- IOUZALEN, NATHALIE ET AL: "LAP, a lymphocyte activation gene-3 (LAG-3)-associated protein that binds to a repeated EP motif in the intracellular region of LAG-3, may participate in the down-regulation of the CD3/TCR activation pathway" EUROPEAN JOURNAL OF IMMUNOLOGY (2001), 31(10), 2885-2891 , 2001 - October 2001 (2001-10), XP001094983

## Description

The present invention relates to molecules binding to specific targets comprising Glu-Pro (EP) repeated motifs such, for example the lymphocyte activation gene-3 (lag-3)-associated protein hereafter named LAP . The invention relates too, to therapeutical compositions containing said molecules, to antibodies directed against said molecules, to therapeutical compositions containing them . Also, the invention relates to methods for screening drugs useful for the treatment of immune disorders.

In previous work, the applicant showed that both LAG-3 and MHC class II were present in the cell fraction of glycosphingolipid-rich complexes (GSL complexes) before the assembly of the immunological synapse by CD3/TCR complex crosslinking.

Using the LAG-3 intracytoplasmic region as bait in the yeast two-hybrid cloning system, applicant have now identified a novel interaction between a new human protein termed LAP for LAG-3-Associated Protein and EP repeated motifs present in LAG-3.

The applicant shows that LAP binds specifically *in vitro* and *in vivo* to the Glu-Pro (EP) repeated motif present in the LAG-3 intracytoplasmic region and that LAP also binds to the EP motif of another functionally important receptor, the PDGFR.

The applicant shows that such an interaction plays an important role in T cell function and homeostasis because LAG-3 acts as a negative regulator of activated T-cells and plays an important role in regulating the expansion of activated T-cells and limiting antigen induced cell death.

LAG-3 associates with the TCR:CD3 complex and interferes with TCR signalling. This down regulation may be activated by disrupting CD4 and CD8 co-receptor function since LAG-3 is expressed on both CD4⁺ and CD8⁺ cells and has been shown to be associated with CD4 and CD4 in raft microdomains.

The LAP protein is likely to transduce the appropriate signals that lead to this control on T cell function and CD4 and CD8 T cell subpopulation homeostasis.

This negative control on T cell activation is of prime importance for the regulation of primary activated T-cells as well as the regulation of T-cell memory development and homeostasis.

LAP protein is encoded by a 1.8 kb RNA message in lymphocytes that is derived from a rare mRNA and encodes a 45 kDa protein that is expressed in most tissues.

Thus, molecules that, as LAP, bind to the EP motif are candidates molecules for a new type of signal transduction and/or coupling of clustered rafts to the microtubule networks that could explain how negative signalling of co-receptors may occur through molecules devoid of any immunoreceptor tyrosine-based inhibitory motifs (ITIM) consensus sequence.

The work conducted by the applicant allow him to verify that supramolecular assemblies between LAG-3, CD3, CD8 and MHC class II molecules result from the organization within raft microdomains (Hannier, S. and Triebel, F., The MHC class II ligand LAG-3 is co-distributed with CD8 and CD3/TCR molecules after their engagement by mAbs or peptide/MHC class I complexes, Int. Immunol. 1999. 11: 1745-1752).

To investigate the pathway involved in LAG-3-dependent TCR signalling regulation, applicant directly clone proteins expressed in activated T cells that would specifically bind to the IC region of hLAG-3.

Using the yeast two-hybrid system and the LAG-3 IC region as bait, the applicant identified a novel protein, termed LAP for LAG-3-associated protein that binds to the Glu-Pro (EP) repeated motifs present within the LAG-3 IC region C-terminus.

These Glu-Pro (EP) repeated motif are present, for example, in the LAG-3 intracytoplasmic region and in the functionally important receptor named Platelet Derived Growth Factor Receptor (PDGFR). Other intracellular signalling molecules including this unusual EP motif are SPY75 and lckBP1, the mouse homologues of the human HS1 product. These molecules have been shown to be involved in TCR signalling.

Then, the present invention relates to a polypeptide binding to a target comprising an EP motif, in particular, to molecules binding to a target comprising an EP motif having the following sequence
[X-(EP)ₙ-Y-(EP)ₘ-Z]ₚ
wherein X, Y and Z, identical or different comprise a sequence of 0 to 10 aminoacids, identical or different, n, m are integers comprised between 0 to 20,

preferably between 3 to 10 at least one from n or m being different from 0 and p is an integer comprised between 1 and 10, said polypeptide consisting of the amino acids sequence identified by SEQ ID No.:1 or a fragment thereof which maintains the binding capacity to EP motifs.

In a embodiment the invention relates to a polypeptide which binds to an EP motif selected from the group comprising the following formula: EPEPEPEPEPEPEPEPEP (SEQ ID N° 3), EPEPEPQLEPEP (SEQ ID N° 4), EPQDEPPEPQLELQVEPEPELEQ (SEQ ID N° 5), or EPEPEPEPEPEP (SEQ ID N° 6).

In another particular embodiment the invention relates to a polypeptide that binds to an aminoacid sequence comprising at least 5 EP motifs over a 19 aminoacid length segment.

Preferably the polypeptide of the invention is a purified polypeptide consisting of the carboxy-terminal amino acids sequence of LAP identified by SEQ ID No.:2 or a fragment thereof which maintains the binding capacity to EP motifs.

An homologous polypeptide relates to a polypeptide which can differ by one or a few amino acid residues when compared with the polypeptide of the invention, as the polypeptides identified by SEQ ID No. :1 or SEQ ID No. :2, but that maintain all the biological functions of said polypeptide, namely, his capacity to bind glu-pro motifs.
- a polypeptide fragment relates to any amino acid sequence contained in the sequence of the polypeptide of the invention, which maintains the binding capacity for at Glu-Pro motifs,
- a polypeptide derivative relates to said entire or fragment polypeptides, labelled with chemical or biological entities in order to be easily detected. Chemical or biological entities may be enzymes, fluorescent labels, coloured particles, etc.

The invention also relates to a nucleic acid molecule consisting of or comprising a polynucleotide sequence coding a polypeptide according to the invention and identified by SEQ ID No.:8.

The invention relates also to an expression vector comprising a nucleic acid molecule according to invention.

For the purpose of the present invention, an "expression vector" refers to any replicable DNA construct used either to amplify or to express DNA, which encodes one of the polypeptides of the invention.

The invention also relates to a host cell transformed with an expression vector according to invention.

Host cells may be prokaryotic or eukaryotic, including but not limited to bacteria, yeasts, insect cells, mammalian cells, including cell lines, which are commercially available.

The invention is also directed to a process for the manufacturing of a purified polypeptide according to the invention, comprising :
a)the transfection of a host cell with an expression vector according to the invention to obtain the expression of the polypeptide,
b)the isolation and purification of the polypeptide from the transfected host cell.

Purification of said polypeptide may be accomplished by any standard methods for purification of membrane or soluble proteins

The invention is also relating to a pharmaceutical composition comprising as active agent at least one polypeptide according to the invention.

The pharmaceutical compositions of the invention are useful for treating immune-related pathologies and in particular they are useful for modulating the immune response.

In a particular embodiment, the pharmaceutical compositions of the invention are useful to enhance the development of CD4 or CD8 T-cell populations.

In another particular embodiment, the pharmaceutical compositions of the present invention are also useful to suppress the development of CD4 or CD8 T-cell populations.

The pharmaceutical composition may comprise as active agent a LAP agonist.

The pharmaceutical composition may also comprise as active agent a LAP antagonist.

A LAP agonist is any molecule that mimics the effect of LAP binding when it binds to the target EP motifs and a LAP antagonist is any molecule that inhibits the effect of LAP binding when it binds to the target EP motif.

The disclosure also includes the use of a polypeptide according to invention for the manufacture of a pharmaceutical composition useful for treating immune-related pathologies or for modulating the immune response

Also disclosed is the use of a polypeptide according to invention for the manufacture of a pharmaceutical composition enhancing the development of CD4 or CD8 T-cell populations.

Also disclosed is the use of a polypeptide according to invention for the manufacture of a pharmaceutical composition suppressing the development of CD4 or CD8 T-cell populations

A molecule that may be used to prepare pharmaceutical compositions is a LAP agonist.

Another molecule that may be used to prepare pharmaceutical compositions is a LAP antagonist

The invention also includes a method for screening drugs comprising the steps of :
- put in contact the drug candidate with a molecule according to the invention in the presence of her target EP motif,
- measure the resulting binding of said molecule to her target.

The method for screening drugs according to the invention allows the screening of drugs selected from the group comprising drugs able to activate T-cell, drugs enhancing the development of CD4 or CD8 T-cell populations, drugs suppressing the development of CD4 or CD8 T-cell populations, drugs active in platelet activation

Preferably the molecule according to the invention to put in contact with the drug candidate in the screening method is a LAP polypeptide.

The invention also relates to antibodies directed to a specific epitope of the polypeptide identified by SEQ ID NO:1.

In particular embodiments, antibodies according to invention are monoclonal antibodies or polyclonal antibodies or Fab, Fab', F(ab') or Fv fragments thereof.

The scope of the invention also comprises a monoclonal or polyclonal antibody or a monoclonal or polyclonal antibody fragments or derivatives which specifically binds a peptide of SEQ ID NO:1, said monoclonal or polyclonal antibody derivative being selected from the group consisting of a monoclonal or polyclonal antibody conjugated to a cytotoxic agent or a radioisotope, and Fab, Fab' or F(ab')₂ fragments of said monoclonal or polyclonal antibody conjugated to a cytotoxic agent or radioisotope.

Antibody fragments are regions from said polyclonal or monoclonal antibodies sequences recognising at least one epitope present in the peptide of SEQ ID NO:1, which maintain the binding capacity for at least one of, said epitopes.

Antibody derivatives are entire or fragment antibodies labelled with chemical or biological entities in order to be easily detected. Chemical or biological entities may be enzymes, fluorescent labels, coloured particles, etc.

The invention relates also to a hybridoma cell line producing a monoclonal antibody according to the invention.

The present invention directs also to a therapeutic composition comprising as active ingredient an antibody according to the invention.

The present invention is also relating to the use of said antibodies in a method for purifying, identifying or quantifying a polypeptide defined in claim 1 or its homologous.

The present invention is also relating to the use of said antibodies to screen drugs, compounds active in intracellular signalling mediated by cell surface receptor,

compounds active in T-cell activation or the regulation of the expansion of activated T-cells, and

compounds active in platelet activation may be screened by using the antibodies of the present invention.

Said antibodies may also be used for the manufacture of a therapeutic composition useful for treating immune-related pathologies.

The present invention is also relating to the use of said antibodies for the manufacture of an immunomodulatory pharmaceutical composition.

The present invention will be comprised in lecture of following experimental results and in figures in annexe where:
Figure 1 represents the *in vitro* interaction of human LAP with hLAG-3.
Figure 1A shows that LAP binds specifically to the natural hLAG-3 (70 kDa) protein present in whole cell lysate of PHA-activated human PBMCs
Figure 1B shows that LAP binds specifically to a protein produced by in vitro translation of an hLAG-3 mRNA in a rabbit reticulocyte lysate.
Figure 2 illustrates interactions tested in the two-hybrid system using co-transformation with two plasmids and mating of two yeast strains.
Figure 2A shows three partial LAP proteins (D1, D2 and D3) lacking their C-terminal domain were cloned in frame with the GAL4 AD protein, using a partial 1104 bp LAP cDNA.
Figure 2B shows that the EP-rich C-terminal region of the PDGF receptor (PDGFR) was fused with the LexA BD.
Figure 2C shows interactions in the two-hybrid system.
Figure 3 represents Western blots autoradiograms obtained with the anti-LAP immune serum, revealing a specific band at 45 kDa.

Western blots were performed using 10 µl total cell lysates of PBMC (lanes 2, 4, 6) or PHA blasts (lanes 1, 3, 5). The blots were incubated in rabbit preimmune serum (lanes 1, 2), rabbit polyclonal antibody against LAP (lanes 3, 4) or the latter preincubated with 10-6 M LAP peptide (lanes 5, 6). The arrow indicates the LAP 45 kDa protein.

Results and discussion:

### 2.1 LAG-3 and MHC class II are expressed in GSL complexes on the surface of human activated T cells

GSL complexes (raft microdomains) were isolated in a low-density fraction, at the interface between the 35% and 5% fractions of a discontinuous sucrose gradient, as described by Montixi et al. (Montixi, C., Langlet, C., Bernard, A. M., Thimonier, J., Dubois, C., Wurbel, M. A., Chauvin, J. P., Pierres, M. and He, H. T., Engagement of T cell receptor triggers its recruitment to low-density detergent-insoluble membrane domains The EMBO Journal 1998. 17: 5334-5348). Twelve fractions of the gradient were analysed by Western-blotting. LAG-3, DR-α as well as p56lck were detected in fraction 9, representing the GSL complex isolates, and were not detected anymore following addition of 0.2 % saponin (cholesterol depletion leading to raft disruption) to 1 % Triton X-100 (data not shown). CD45, a phosphotyrosine phosphatase known to be excluded from raft microdomains was used as a negative control. Thus, LAG-3 is present in raft microdomains before engagement of the TCR by specific mAb or peptide/MHC complexes.

In addition, it was found that MHC class II (DR-α) molecules were also present in raft microdomains on activated T cells (results not shown). The partitioning of MHC class II into the raft fraction has been reported to occur in the myelomonocytic THP-1 cells following their crosslinking with antibodies and to be mandatory for protein tyrosine kinase (PTK) activation (Huby, R. D. J., Dearman, R. J. and Kimber, I., Intracellular phosphotyrosine induction by major histocompatibility complex class II requires co-aggregation with membrane rafts J. Biol. Chem. 1999. 274: 22591-22596). In B cells, MHC class II were found to be constitutively present in rafts and this concentration of MHC class II molecules facilitates antigen presentation (Anderson, H. A., Hiltbold, E. M. and Roche, P. A., Concentration of MHC class II molecules in lipid rafts facilitates antigen presentation Nature Immunol. 2000. 1: 156-162).

The presence of LAG-3 in raft microdomains before engagement of the TCR argues for its close association with CD3/TCR complexes and explains, in part, previous observations where LAG-3 was found to be coclustered with CD3/TCR complexes and also with CD8 in cocapping experiments (Hannier, S. and Triebel, F., The MHC class II ligand LAG-3 is co-distributed with CD8 and CD3/TCR molecules after their engagement by mAbs or peptide/MHC class I complexes Int. Immunol. 1999. 11: 1745-1752).

### 2.2 Isolation of a novel human protein, LAP, interacting with LAG-3

An interaction screening was performed by using the yeast two-hybrid system to identify proteins that bind to the intracellular domain of human LAG-3 *in vivo.* First, it was verified that no LAG-3 construct in pLex or pLex/NLS displayed any lacZ reporter gene activity in yeast cells expressing pGAD without insert. This indicated that LAG-3 does not show any non-specific binding to DNA sequences leading to GAL promoter activation. Then, strain L40 was transformed with pLex/NLS-hLAG-3/I to screen about 2 x 10⁵ colonies of the human activated T-cell cDNA library. Around 200 colonies that grew on histidine-free drop-out medium were selected, replaced onto selective medium and assayed for β-galactosidase expression. From these, 13 showed reporter gene activities. In order to confirm the specificity of these interactions, the plasmid DNA from selected clones was isolated and used for transformation of the strain AMR70, which were then mated with strain L40 containing either the bait plasmid pLex/NLS-hLAG-3/I or a control plasmid (pLex-Lamin or pLex/NLS-RalB). Three specific clones were obtained showing strong interaction with hLAG-3/I (signals appeared in less than 2 hrs) and not with Lamin or RalB. The inserts of these clones were submitted to restriction mapping and sequence analysis. The three cDNAs were found to encode a unique partial (i.e. lacking the ATG translation initiation codon) sequence of 243 amino acids, termed LAP (not shown). This novel molecule has some homology with the C terminal region of the TCP-10 protein previously cloned in human (Islam, S. D., Pilder, S. H., Decker, C. L., Cebra-Thomas, J. A. and Silver, L. M., The human homolog of a candidate mouse t complex responder gene : conserved motifs and evolution with punctuated equilibria, Human Molecular Genetics 1993. 2: 2075-2079 and Bibbins, K. B., Tsai, J. Y., Schimenti, J., Sarvetnick, N., Zoghbi, H. Y., Goodfellow, P. and Silver, L. M., Human homologs of two testes-expressed loci on mouse chromosome 17 map to opposite arms of chromosome 6, Genomics 1989. 5: 139-143) and mouse (Schimenti, J., Cebra-Thomas, J. A., Decker, C. L., Islam, S. D., Pilder, S. H. and Silver, L. M., A candidate gene family for the mouse t complex responder (Tcr) locus responsible for haploid effects on sperm function, Cell 1988. 55: 71-78; Ewulonu, U. K., Snyder, L., Silver, L. M. and Schimenti, J. C., Promoter mapping of the mouse Tcp-10bt gene in transgenic mice identifies essential male germ cell regulatory sequences, Molecular Reproduction and Development 1996. 43: 290-297 and Cebra-Thomas, J. A., Decker, C. L., Snyder, L. C., Pilder, S. H. and Silver, L. M., Allele- and haploid-specific product generated by alternative splicing from a mouse t complex responder locus candidate, Nature 1991. 349: 239-241) *TCP-10* is a T-complex responder (TCP) gene that may play a role in the transmission ratio distortion phenotype. A region of LAP is 56% identical to the 181 C-terminal residues of human TCP-10 protein and 66% identical to the 106 C-terminal residues of the murine TCP-10 protein.

The 5' end of the LAP cDNA was further extended by 5'RACE cloning starting from PHA-blasts mRNA. Analysis of the LAP cDNA revealed a nucleotide sequence of 1353 bases that contains a single open reading frame (ORF) of 372 amino acids. This ORF starts at position 70 and ends with the translation stop codon, TGA, located at nt 1186.

It was found that this LAP sequence is in 99% identical (9 nt mismatches including 4 in the coding region with a single a.a. difference at the carboxy-terminus) to the 3'end of the recently published CPAP (centrosomal P4.1-associated protein) molecule, which is part of the γ-tubulin complex (Hung, L. Y., Tang, C. C. and Tang, T. K., Protein 4.1 R-135 interacts with a novel centrosomal protein (CPAP) which is associated with the gamma-tubulin complex, Mol. Cell. Biol. 2000. 20: 7813-7825). These 9 nt mismatches were also found on several EST sequences, confirming the differences observed between CPAP and LAP. (Hung, L. Y., et al. Mol. Cell. Biol. 2000. 20: 7813-7825).

Sequence identity of TCP-10, CPAP and LAP proteins is restricted to two conserved regions at the COOH-terminus. One carries a leucine zipper, which may form a series of heptads. Repeats involved in coiled-coil formations, and the second contains unusual glycine repeats (Hung, L. Y., et al. Mol. Cell. Biol. 2000. 20: 7813-7825).

Additional tests were performed in order to verify whether human LAP could bind to the murine LAG-3 IC region. It was observed a weak interaction between these two heterologous proteins with a small activation of the HIS3 gene but no detectable LacZ activity. Next, it was examined which region of human LAG-3 interacts with LAP. The binding of LAP with hLAG-3/IΔC and hLAG-3/EP constructs was tested in yeast cells and it was found that LAP indeed binds specifically with the short C-terminal region of LAG-3 containing the EP-rich region. These results illustrating interaction of LAP and LAG-3 proteins (a) are shown in table 1 hereafter.

**Table 1**

| fused to LexA BD | | | fused to Gal4 AD | | |
|---|---|---|---|---|---|
| | LAG-3 regions | - | Lamin | RalB | LAP |
| hLAG-3/I | R457 to L503 | - | - | - | ++++ |
| NLS-hLAG-3/I | R457 to L503 | - | - | - | +++++ |
| NLS-mLAG-3/I | L456 to L507 | - | - | - | + |
| NLS-hLAG-3/I?C | R457 to E481 | - | - | - | +/- |
| hLAG-3/EP | E478 to L503 | - | - | - | ++ |
| NLS/hLAG-3/EP | E478 to L503 | - | - | - | ++++ |

Where LAG proteins are hLAG-3 and mLAG-3, IC regions were expressed as fusion proteins to the LexA DNA binding domain (LexA BD) in the pLex vector containing or not a nuclear localization sequence (NLS). The pGAD vector encoded the GAL4 activation domain (GAL4 AD) alone or fused to LAP or an unrelated protein (Lamin or RalB). Two procedures for interaction studies were performed: (i) co-transfection of yeast strain L40 with the two indicated plasmid combinations shown, (ii) transformation of strain L40 with a pLex construct which are then mated with strain AMR70 transformed with a pGAD construct.

A demonstration of *in vitro* binding between LAP and hLAG-3 proteins, LAP linked to GST or GST alone were expressed in bacteria and bound to glutathione-Sepharose beads was performed as described hereafter.

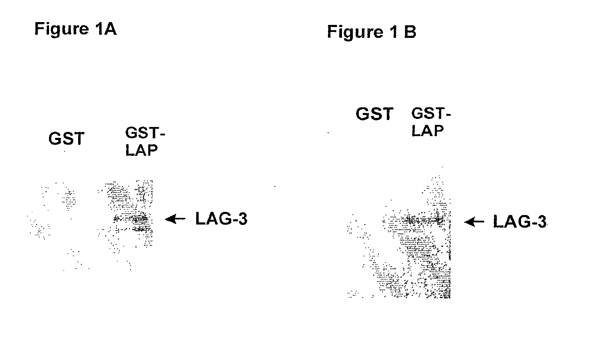

Bound proteins were incubated with total cell lysates prepared from PHA-activated T lymphocytes. The results demonstrate that the LAG-3 protein was specifically precipitated from the T-cell lysate when using affinity beads containing the LAP protein (Fig. 1A). The control GST beads did not precipitate any detectable LAG-3 protein from the T-cell lysate. Therefore, LAG-3 binds specifically to the LAP protein *in vitro,* in agreement with the data obtained from the yeast two-hybrid screening procedure.

In order to verify that the interaction between LAP and LAG-3 proteins in both the yeast two-hybrid system and in T-cell lysates does not require an additional adaptor protein, a direct binding assay in which the *in vitro*-translated LAG-3 protein was tested for interaction with beads bound to GST-LAP or GST alone was performed.

As shown in Figure 1B, affinity beads containing the GST-LAP fusion protein pulled down the LAG-3 protein in a specific manner. This supports the existence of a specific direct physical interaction between LAP and LAG-3 proteins without the need for the presence of a third adaptor protein.

Overall, the interaction between LAP and hLAG-3 has been confirmed both *in vivo* and *in vitro* using recombinant LAP protein. In particular, it was showed that the LAP protein was able to bind LAG-3 in lysates of activated T cells. This interaction was specific and was also observed vice versa using *in vitro* translated recombinant LAG-3.

### 2.3 The C-terminus region of LAP binds the EP region of hLAG-3.

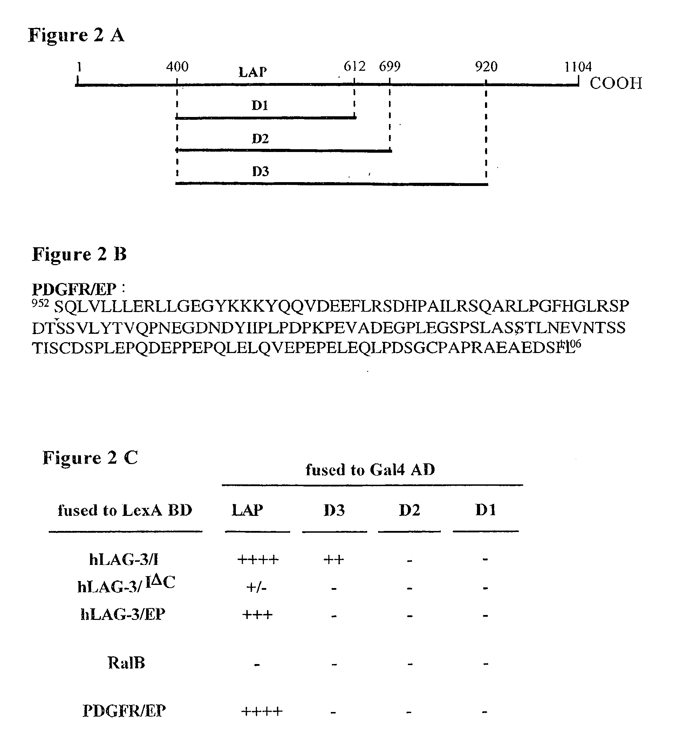

To determine the region of the LAP protein that contains the LAG-3 binding site, deletion mutants of the LAP cDNA was constructed (Fig. 2A). The binding of these mutants with hLAG-3/I, hLAG-3/IAC and hLAG-3/EP were tested, with Ral B as a negative control. Deletion of the extreme C-terminal regions (mutant D3) already abolished some binding activity (Fig. 2C), while the shorter constructs (D1 & D2) did not bind to hLAG-3 at all.

Thus, the binding site for LAP on the EP motifs is located in its C-terminal region.

LAP would then function to cluster rafts into the immunological synapse following TCR engagement, a phenomenon that requires the polarization of actin and microtubules (Simons, K. and Toomre, D., Lipid rafts and signal transduction Nature 2000. 1: 31-39).

### 2.3 LAP binds to the intracytoplasmic region of the PDGF receptor containing an EP motif

The PDGF receptor (Claesson-welsh, L., A.Eriksson, A.Morén, L.Severinsson, B.Ek, A.Ostman, C.Betsholtz and C.H.Heldin, cDNA cloning and expression of a human platelet-derived growth factor (PDGF) receptor specific for B-chain-containing PDGF molecules, Mol. Cell. Biol. 1988. 8: 3476-3486) has a long intracytoplasmic tail containing numerous motifs known to be involved in signalling. It was noticed that a repeated EP motif not known to be involved in transduction signalling was found in its C-terminal region (Figure 2B).

Surprisingly the LAP protein could bind to this EP motif-containing segment.

Thus, LAP interactions with other membrane receptor intracytoplasmic regions containing the EP motif have crearly been identified, since the present work shows that it binds to the PDGFR intracellular region in addition to hLAG-3 and mLAG-3.

Thus, this EP motif appears as a common transduction motif, that could be used by other functionally important receptors.

### 2.4 LAP is a 45 kDa protein expressed in all tested human cells

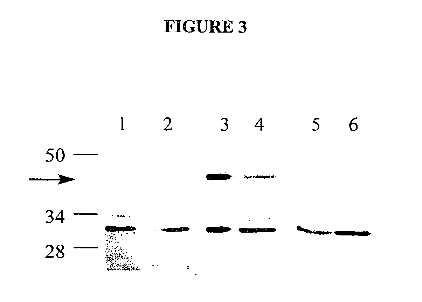

To determine the size and expression of the LAP protein, total cell lysates were analyzed by Western blotting with a rabbit polyclonal serum rose against a LAP peptide with no sequence homology with TCP-10. Two bands at 30- and 45 kDa were detected in PBMCs on activated T-cells (Figure 3). The 30 kDa band was shown to be non-specific since it was also detected using the preimmune serum (Fig. 3). The 45 kDa band corresponds to LAP as it was no longer detected following pre-incubation of the immune serum containing the LAP peptide (10⁻⁶ M at 4°C for 1 hr) (Figure 3) while pre-incubation with a control peptide had no effect (data not shown). In addition, this 45 kDa band was found in cytoplasmic but not in nucleic T cell extracts (data not shown).

These results clearly indicate that LAP is expressed as a 45 kDa cytoplasmic protein in PBMCs and in activated T cells with a higher expression level in the latter cells.

Western blotting was also performed with total cell lysates of the Jurkat T cell line, two EBV-transformed B cell lines and a renal cell carcinoma cell line (RCC7).

LAP is also expressed in these cell lines as a 45 kDa protein with lower expression in PBMC (data not shown). LAP is thus expressed in T and non-T hematopoietic cell lines as well as in non-hematopoietic cell lines.

In addition, LAP was detected in different untransformed human tissues, including the lung, liver, kidney, testes (no overexpression, in contrast to CPAP), pancreas and heart, but not in the spleen and brain (data not shown).

### 2.5 Two RNA species are derived from the LAP gene.

The LAP gene was first analysed by digesting DNA from different cell lines and PBLs, Southern blotting and hybridizing using the LAP cDNA as a probe. Unique EcoRI (5.5 kb), Hind III (9 kb) and Xho I (>12 kb) fragments were found indicating that the LAP or CPAP gene is either present in the human genome as a single copy gene or represents two closely related genes (data not shown).

Total and poly-A⁺ RNA samples of PHA-blasts were run on a denaturing agarose gel and analyzed by Northern blotting. The LAP RNA seemed to be rarely expressed, as it was only detected by using 15 µg of poly-A⁺ RNA while not being detected in total RNA samples (up to 20 µg, data not shown). Two faint bands hybridised with the labelled cDNA LAP probe, one with a size of 4.5 kb and a weaker one at 1.8 kb. As these two bands correspond exactly to the sizes of the 28S and 18S rRNA, the blot was then rehybridized with saturating amounts of ribosomal RNA (10 µg/ml) added to prevent any non-specific binding of the probe to the remaining rRNA in the sample and the same result was obtained (data not shown). Since these two signals were only seen with highly purified poly-A⁺ RNA and not with total RNA samples containing a greater amount of rRNA, we concluded that LAP was specifically expressed as a 1.8 kb mRNA. The stronger 4.5 kb signal may correspond to CPAP, which has been shown to be weakly expressed in most tissues, except testis (Hung, L. Y., Tang, C. C. and Tang, T. K., Protein 4.1 R-135 interacts with a novel centrosomal protein (CPAP) which is associated with the gamma-tubulin complex, Mol. Cell. Biol. 2000. 20: 7813-7825).

Thus, LAP is a new human protein, expressed in all tested human cells and derived from a rare mRNA. It appears that LAP and CPAP are derived from either a single gene or two closely related genes which are strongly expressed in the testes for the CPAP mRNA (4.5 kb) and weakly expressed in other cells as two messages (4.5 kb and 1.8 kb) coding for CPAP and LAP, respectively.

The specific immunoprecipitation of LAG-3 by LAP-GST beads from activated T lymphocyte lysates indicates that the two overlapping 150 kDa CPAP (Hung, L. Y., Tang, C. C. and Tang, T. K., Protein 4.1 R-135 interacts with a novel centrosomal protein (CPAP) which is associated with the gamma-tubulin complex, Mol. Cell. Biol. 2000. 20: 7813-7825) and 45 kDa LAP proteins have different functions, that is binding to γ-tubulin in the centrosome especially in testis cells (CPAP) or binding to EP motifs present on membrane expressed receptors (LAP).

EP motifs are rare in human proteins, and the specific binding of LAP on such motifs has important biological significance for signal transduction and/or coupling of clustered rafts to the microtubule networks.

### 3 Materials and methods

### 3.1 Plasmid construction

The hLAG-3/I and mLAG-3/I fragments encode the full length intracellular region of human LAG-3 and murine LAG-3, respectively. The hLAG-3/IΔC encodes the intracellular domain of human LAG-3 deleted of its 22 C-terminal amino acids (ΔC) whereas hLAG-3/EP codes only for the EP-rich region located at the end of the C-terminal part of hLAG-3. The PCR products were cloned into the two hybrid vectors pBMT116 (pLex) or a derivative containing an additional Nuclear Localization Sequence (pLex/NLS) (Vojtek, A. B. and Hollenberg, S. M., Ras-Raf interaction : two-hybrid analysis, Methods Enzymol. 1995. 255: 331-342) in frame with the LexA DNA binding protein yielding the following constructs:
- pLex-hLAG-3/I and pLex/NLS-hLAG-3/I (from R⁴⁵⁷ to L⁵⁰³)
- pLex/NLS-mLAG-3 (from L⁴⁵⁶ to L⁵⁰⁷)
- pLex-hLAG-3/IΔC and pLex/NLS-hLAG-3/IΔC (from R⁴⁵⁷ to E⁴⁸¹)
- pLex-hLAG-3/EP and pLex/NLS-hLAG-3/EP (from E⁴⁷⁸ to L⁵⁰³).

### 3.2 Two-hybrid screen and interaction analysis

Yeast, medium and two-hybrid procedures were handled according to published methods (Vojtek, A. B. and Hollenberg, S. M., Ras-Raf interaction : two-hybrid analysis, Methods Enzymol. 1995. 255: 331-342; Kaiser, C., Michaelis, S. and Mitchell, A., Methods in yeast genetics Cold Spring Harbor Laboratory 1994). For the two hybrid-screen, it was used a human activated PBL library cloned in the pGAD-1318 vector (Hybrigenics, Paris, France) which contains the activation domain of GAL4 under the control of the entire ADH1 strong yeast promoter. For library screening, yeast strain L40 which contains the LacZ and HIS3 reporter genes downstream of the binding sequence of LexA, was sequentially transformed with pLex/NLS-hLAG-3/I and 60 µg of the human activated T cell library using the lithium acetate method. Double transformants were plated on yeast drop-out medium lacking tryptophan, leucine and histidine, and were incubated at 30°C for 3 days. Positive colonies His⁺ were patched on selective plates for growth and were then replicated on Whatman 40 paper. The β-galactosidase activity was tested by a filter assay.

For interaction studies, two methods were used : by co-transformation of strain L40 with pairs of pLex and pGAD vectors, or by mating the strain L40 expressing a pLex vector with the strain AMR70 containing a pGAD vector. In both cases, binding was tested for growth in histidine-deficient medium and for β-galactosidase activity. Signals described as being negative were not detected even after 3 days or 24 hrs for the HIS3 and LacZ reporter genes, respectively. No discrepancy was ever observed between the histidine auxotrophy and the β-galactosidase tests.

### 3.3 Protein expression and purification

LAP polypeptide was expressed as a glutathione S-transferase (GST) fusion protein in *Escherichia coli* and immobilized on affinity matrix beads. Briefly, fresh overnight cultures of *E. Coli* HB101 or XL-1 blue cells harboring the pGEX plasmid expressing GST or GST-LAP proteins were diluted 1 :10 in Luria-Bertani (LB) broth supplemented with 20 µg/ml ampicillin and the cultures were grown for 3 h with 0.1 mM IPTG (Sigma, St. Louis, MO). Cell pellets were collected by centrifugation and lysed in Tris buffer containing 1% NP-40 and anti-proteases. The soluble fraction was prepared by centrifugation at 10,000 g for 15 min at 4°C. The GST and recombinant GST fusion proteins were purified by coupling to Glutathione Sepharose 4B beads (Pharmacia, Uppsala, Sweden) by gentle mixing at 4°C for 40 min followed by extensive washing. The protein-bound affinity beads were analyzed and quantitated by Coomassie blue R-250 staining following SDS-PAGE analysis.

### 3.4 Preparation of cell lysates and in vitro binding assays

Human PBMCs were isolated from venous blood by Ficoll-Paque density gradient centrifugation. T lymphocytes were obtained by stimulating PBMCs with 1 µg/ml of PHA-P (Wellcome, Beckenham, UK) at 37°C and 10% CO₂ in complete culture medium (RPMI 1640 supplemented with 10% heat inactivated human AB serum, 4 mM L-glutamine, 1 mM pyruvate, 0.2 mM NaOH, 50,000 IU penicillin and 50 mg/ml streptomycin). After 3 days of culture, whole cell lysates were prepared in Tris cell lysis buffer containing 1% NP-40 and anti-proteases.

The hLAG-3 protein was synthesized *in vitro* using the T7-coupled rabbit reticulocyte lysate system (TNT, Promega, Madison, WI). Equal amounts of GST-LAP or control GST proteins immobilized on beads were incubated for 3 hrs at 4°C with direct whole cell lysates (after centrifugation of nuclei) or with the *in vitro* translated hLAG-3 protein in a binding buffer (20 mM Tris-HCl pH 7.5, 50 mM NaCl, 1 mM PMSF, 1 µg/ml leupeptin, 1 µg/ml aprotinin). Bound proteins were then extensively washed in PBS buffer and analyzed by Western blotting.

### 3.5 Cell lines and antibodies

The Jurkat T cell line and the Epstein Barr Virus (EBV)-transformed B cell line were grown in complete 1640 RPMI culture medium at 37°C and 6 % CO₂. RCC7 (a renal cell carcinoma cell line, (Gaudin, C., Kremer, F., Angevin, E., Scott, V. and Triebel, F., A HSP70-2 mutation recognized by cytolytic T lymphocytes on a human renal cell carcinoma, J. Immunol. 1999. 162: 1730-1738) were cultivated in complete DMEM medium at 37°C and 6 % CO₂.

A polyclonal serum was raised against a peptide (SPREPLEPLNFPDPEYK) derived from the deduced amino-acid sequence of LAP by immunizing rabbits with three injections of peptide-BSA (Neosystem, Strasbourg, France).

### 3.6 Western blot

10⁶ cells were washed and lysed at 4°C for 60 min in 100 µl Tris cell lysis buffer. Cell debris were removed by 10 min centrifugation at 10,000 g and the lysates heat-denaturated in SDS sample buffer for 5 min. Total cell lysates were separated by SDS-PAGE and transferred to nitrocellulose membranes. Membranes were saturated with 5% dry milk for 1 hr at 37°C and incubated with primary antibody diluted 1:3000 in TBS for 1.5 hr with slow agitation. After incubating the membranes with the GAR-peroxidase secondary antibody, the signal was detected by enhanced chemiluminescence (ECL, Amersham, Buckinghamshire, UK). To determine the tissue distribution of LAP, a commercial Western blot containing 75 µg of total cellular protein from eight different human tissues (Chemicon, Temecula, USA) was used.

## Claims

1. A polypeptide binding to a target comprising a Glu-Pro (EP) motif having the following sequence :
[X-(EP)ₙ-Y-(EP)ₘ-Z]ₚ
wherein X, Y and Z, identical or different comprise a sequence of 0 to 10 aminoacids, identical or different, n, m are integers comprised between 0 to 20, preferably between 3 to 10 at least one from n or m being different from 0 and p is an integer comprised between 1 and 10,
said polypeptide consisting of the amino acids sequence of LAP identified by the sequence SEQ ID NO: 1, or a fragment thereof which maintains the binding capacity to EP motifs.

2. A polypeptide according to claim 1 consisting of the carboxy terminal amino acids sequence of LAP identified by the sequence SEQ ID NO: 2, or a fragment thereof which maintains the binding capacity to EP motifs.

3. A polypeptide according to either of claim 1 or 2 which binds to an EP motif selected from the group comprising the following formula: EPEPEPEPEPEPEPEPEP (SEQ ID NO: 3), EPEPEPQLEPEP (SEQ ID NO: 4), EPQDEPPEPQLELQVEPEPELEQ (SEQ ID NO: 5), or EPEPEPEPEPEP (SEQ ID NO: 6).

4. A polypeptide according to either of claim 1 or 2 which binds to an amino acids sequence comprising at least 5 EP motifs over a 19 amino acids segment.

5. A nucleic acid molecule comprising a polynucleotide sequence coding a polypeptide according to any of claims 1 to 4 and identified by the sequence SEQ ID NO: 8.

6. An expression vector comprising a nucleic acid molecule according to claim 5.

7. A host cell transformed with an expression vector according to claim 6.

8. Process for the manufacturing of a polypeptide according to claim 1, comprising :
a) the transfection of a host cell with an expression vector according to claim 6 to obtain the expression of the polypeptide;
b) the isolation and purification of the polypeptide from the transfected host cell.

9. A pharmaceutical composition comprising as active agent at least one polypeptide according to any of claims 1 to 4.

10. Use of a polypeptide according to any of claims 1 to 4 for the manufacture of a therapeutical composition suppressing the development of CD4 or CD8 T-cell populations.

11. A method for screening drugs comprising the steps of :
- put in contact, the candidate drug with a polypeptide according to any of claims 1 to 4 in the presence of its target EP motif;
- measure the resulting binding of said polypeptide to its target.

12. A method according to claim 11 wherein said drugs are selected from drugs able to activate T-cell, drugs enhancing the development of CD4 or CD8 T-cell populations, drugs suppressing the development of CD4 or CD8 T-cell populations, drugs active in platelet activation.

13. Antibodies directed to a specific epitope of a polypeptide selected from the group consisting of polypeptides or peptides identified by SEQ ID NO:1 or SEQ ID NO:2.

14. Antibodies according to claim 13 wherein said antibodies are monoclonal antibodies or Fab, Fab', F(ab')₂ or Fv fragments thereof.

15. A monoclonal antibody or monoclonal antibody derivative which specifically binds a peptide selected from the group consisting of polypeptides or peptides identified by SEQ ID NO:1 or SEQ ID NO:2, said monoclonal antibody derivative being selected from the group consisting of a monoclonal antibody conjugated to a cytotoxic agent or a radioisotope, and Fab, Fab', F(ab')₂ or Fv fragments of said monoclonal antibody conjugated to a cytotoxic agent or radioisotope.

16. A hybridoma cell line producing the monoclonal antibody of claim 15.

17. A therapeutic composition comprising as active ingredient an antibody according to any of claims 13 to 15.

18. Use of antibodies according to claims 13 to 15. in a method for purifying, identifying or quantifying a polypeptide according to any of claims 1 to 4.

19. Use of antibodies according to any of claims 13 to 15 to screen drugs.

## Patentansprüche

1. Eine Polypeptid, das an ein Ziel bindet, das ein Glu-Pro (EP)- Motiv enthält, das folgende Sequenz hat:
[X-(EP)ₙ-Y-(P)ₘ-Z]ₚ
wobei identische oder unterschiedliche X, Y und z eine Sequenz von 0 bis 10 gleichen oder unterschiedlichen Aminosäuren enthalten, n, m ganze Zahlen zwischen 0 und 20, vorzugsweise zwischen 3 und 10, sind, wobei mindestens ein n oder m ungleich 0 ist und p eine ganze Zahl zwischen 1 und 10 ist,
wobei besagtes Polypeptid aus der durch die Sequenz SEQ ID NO: 1 definierten Aminosäuresequenz von LAP besteht oder eines Fragments hiervon, das die Bindungsfähigkeit an EP-Motive erhält.

2. Ein Polypeptid gemäß Anspruch 1, das karboxyterminal aus der Aminosäuresequenz von LAP besteht, die durch die Sequenz SEQ ID NO: 2 definiert ist, oder ein Fragment hiervon, das die Bindungsfähigkeit an EP-Motive erhält.

3. Ein Polypeptid gemäß Anspruch 1 oder 2, das an ein EP-Motiv bindet, das innerhalb der Gruppe gewählt wird, die folgende Formel enthält: EPEPEPEPEPEPEPEPEP (SEQ ID NO: 3), EPEPEPQLEPEP (SEQ ID NO: 4), EPQDEPPEPQLELQVEPEPELEQ (SEQ ID NO: 5), oder EPEPEPEPEPEP (SEQ ID NO: 6).

4. Ein Polypeptid gemäß Anspruch 1 oder 2, das an eine Aminosäuresequenz bindet und mindestens 5 EP-Motive innerhalb eines 19 Aminosäuresegments enthält.

5. Ein Nukleinsäuremolekül, das eine Polynukleotidsequenz enthält, die ein Polypeptid gemäß einem beliebigen der Ansprüche 1 bis 4 kodiert und durch die Sequenz SEQ ID NO: 8 definiert ist.

6. Ein Expressionsvektor, der ein Nukleinsäuremolekül gemäß Anspruch 5 enthält.

7. Eine veränderte Wirtszelle mit einen Expressionsvektor gemäß Anspruch 6.

8. Verfahren zur Herstellung eines polypeptids gemäß Anspruch 1, das folgendes enthält :
a) Die Transfektion einer Wirtszelle mit einem Expressionsvektor gemäß Anspruch 6, um die Expression des Polypeptids zu erhalten;
b) Die Isolierung und Reinigung des Polypeptids von der transfektierten wirtszelle.

9. Eine pharmazeutische Zusammensetzung, die als Wirkstoff mindestens ein Polypeptid gemäß einem beliebigen der Ansprüche 1 bis 4 umfasst.

10. Verwendung eines Polypeptids gemäß einem beliebigen der Ansprüche 1 bis 4 zur Herstellung einer therapeutischen Zusammensetzung, die die Entwicklung von CD4 oder CD8 T-Zell-Populationen unterdrückt.

11. Ein Verfahren zum Screening von Medikamenten, das folgende Schritte umfasst:
- das in Kontaktbringen des potentiellen Medikaments mit einem Polypeptid gemäß einem beliebigen der Ansprüche 1 bis 4 bei Vorhandensein seines Ziel-EP-Motivs;
- Messen der erhaltenen Bindung des besagten Polypeptids an sein Ziel.

12. Ein Verfahren gemäß Anspruch 11, wobei besagte Medikamente unter Medikamenten gewählt werden, die T-Zellen aktivieren können, die die Entwicklung von CD4 oder CD8 T-Zell-Populationen verstärken, die Entwicklung von CD4 oder CD8 T-Zell-Populationen unterdrücken, in der Thrombozytenaktivierung aktiv sind.

13. Antikörper, die auf ein spezifisches Epitop eines Polypeptids gerichtet sind, das aus der Gruppe gewählt wird, die aus Polypeptiden oder durch SEQ ID NO:1 oder SEQ ID NO:2 identifizierten Peptiden besteht.

14. Antikörper gemäß Anspruch 13, wobei besagte Antikörper monoklonale Antikörper oder Fab, Fab', F(ab')₂ oder deren Fv Fragmente sind.

15. Ein spezifisch ein Peptid bindender monoklonaler Antikörper oder Derivat eines monoklonalen Antikörpers, innerhalb der Gruppe gewählt, die aus durch SEQ ID NO:1 oder SEQ ID NO:2 identifizierten Peptiden oder Polypeptiden besteht, wobei besagtes Derivat eines monoklonalen Antikörpers innerhalb der Gruppe gewählt wird, die aus einem an ein zytotoxisches Mittel oder Radioisotop konjugierten monoklonalen Antikörper, und Fab, Fab', F(ab')₂ oder Fv Fragmenten des besagten an ein zytotoxisches Mittel oder Radioisotop konjugierten monoklonalen Antikörpers besteht.

16. Eine Hybridoma-zelllinie, die den monoklonalen Antikörper von Anspruch 15 herstellt.

17. Eine therapeutische zusammensetzung, die als Wirkstoff einen Antikörper entsprechend einem beliebigen der Ansprüche 13 bis 15 enthält.

18. Verwendung von Antikörpern gemäß den Ansprüchen 13 bis 15 in einem Verfahren zur Reinigung, Identifizierung oder Quantifizierung eines Polypeptids gemäß einem beibiegen der Ansprüche 1 bis 4.

19. Verwendung von Antikörpern gemäß einem beliebigen der Ansprüche 13 bis 15 zum Screening von Medikamenten.

## Revendications

1. Un polypeptide se liant à une cible comprenant un motif Glu-Pro (EP) ayant la séquence suivante :
[X-(EP)ₙ-Y-(EP)ₘ-Z]ₚ
où X, Y et Z, identiques ou différents comprennent une séquence de 0 à 10 acides aminés, identiques ou différents, n, m sont des nombres entiers compris entre 0 et 20, de préférence entre 3 et 10 au moins un de n ou de m étant différent de 0 et p est un nombre entier compris entre 1 et 10,
ledit polypeptide consistant en la séquence d'acides aminés de LAP identifiée par la séquence SEQ ID NO:1, ou un fragment de celle-ci qui maintient la capacité de liaison aux motifs EP.

2. Un polypeptide selon la revendication 1 consistant en la séquence d'acides aminés carboxy-terminale de LAP identifiée par la séquence SEQ ID NO: 2, ou un fragment de celle-ci qui maintient la capacité de liaison aux motifs EP.

3. Un polypeptide selon l'une quelconque des revendications 1 ou 2 qui se lie à un motif EP choisi parmi le groupe comprenant les formules suivantes: EPEPEPEPEPEPEPEPEP (SEQ ID NO: 3), EPEPEPQLEPEP (SEQ ID NO: 4), EPQDEPPEPQLELQVEPEPELEQ (SEQ ID NO: 5), or EPEPEPEPEPEP (SEQ ID NO: 6).

4. Un polypeptide selon l'une quelconque des revendications 1 ou 2 qui se lie à une séquence d'acides aminés comprenant au moins 5 motifs EP sur un segment de 19 acides aminés.

5. Une molécule d'acide nucléique comprenant une séquence de polynucléotide codant pour un polypeptide selon l'une quelconque des revendications 1 à 4 et identifiée par la séquence SEQ ID NO: 8.

6. Un vecteur d'expression comprenant une molécule d'acide nucléique selon la revendication 5.

7. Une cellules hôte transformée avec un vecteur d'expression selon la revendication 6.

8. Procédé pour la fabrication d'un polypeptide selon la revendication 1 comprenant:
a) la transfection d'une cellule hôte avec un vecteur d'expression selon la revendication 6 pour obtenir l'expression du polypeptide;
b) l'isolement et la purification du polypeptide à partir de la cellule hôte transfectée.

9. Une composition pharmaceutique comprenant comme principe actif au moins un polypeptide selon l'une quelconque des revendications 1 à 4.

10. Utilisation d'un polypeptide selon l'une quelconque des revendications 1 à 4 pour la fabrication d'une composition thérapeutique supprimant le développement des populations de cellules T CD4 ou CD8.

11. Un procédé pour le criblage de médicaments comprenant les étapes de:
- mise en contact du médicament candidat avec un polypeptide selon l'une quelconque revendication 1 à 4 en présence de sa cible à motif EP;
- mesure de la liaison résultant dudit polypeptide à sa cible.

12. Un procédé selon la revendication 11 où lesdits médicaments sont choisis parmi des médicaments capables d'activer les cellules T, des médicaments augmentant le développement des populations de cellules T CD4 ou CD8, des médicaments supprimant le développement des populations de cellules T CD4 ou CD8, des médicaments actifs sur l'activation plaquettaire.

13. Anticorps dirigés contre un épitope spécifique d'un polypeptide choisi parmi le groupe consistant en des polypeptides ou peptides identifiés par SEQ ID NO:1 ou SEQ ID NO: 2.

14. Anticorps selon la revendication 13 où lesdits anticorps sont des anticorps monoclonaux ou Fab, Fab', F(ab')₂ ou des fragments Fv de ceux-ci.

15. Un anticorps monoclonal ou un dérivé d'anticorps monoclonal qui se lie spécifiquement à un peptide choisi parmi le groupe consistant en des polypeptides ou peptides identifiés par SEQ ID NO:1 or SEQ ID NO:2, ledit dérivé d'anticorps monoclonal étant choisi parmi le groupe consistant en un anticorps monoclonal conjugué à un agent cytotoxique ou à un radio-isotope, et Fab, Fab', F(ab')₂ ou des fragments Fv dudit anticorps monoclonal conjugué à un agent cytotoxique ou à un radioisotope.

16. Une lignée cellulaire d'hybridome produisant l'anticorps monoclonal de la revendication 15.

17. Une composition thérapeutique comprenant comme principe actif un anticorps selon l'une quelconque des revendications 13 à 15.

18. Utilisation d'anticorps selon les revendications 13 à 15 dans un procédé pour purifier, identifier ou quantifier un polypeptide selon l'une quelconque des revendications 1 à 4.

19. Utilisation d'anticorps selon l'une quelconque des revendications 13 à 15 pour le criblage de médicaments.


### SEQUENCE LISTING

<110> Institut Gustave-Roussy Université d'Orsay Paris-Sud
<120> MOLECULES BINDING TO GLU-PRO MOTIFS, THERAPEUTICAL COMPOSITIONS CONTAINING THEM AND THEIR APPLICATIONS.
<130> B14692EP-IGR-PS
<140> EP2001/xxxxx
<141> 2001-09-19
<160> 9
<170> PatentIn version 3.0
<210> 1
   <211> 372
   <212> PRT
   <213> Homo sapiens
<220>
   <221> PEPTIDE
   <222> (1)..(372)
   <223> LAP protein
<400> 1
<210> 2
   <211> 135
   <212> PRT
   <213> homo sapiens
<220>
   <221> PEPTIDE
   <222> (1)..(135)
   <223> COOH-terminal peptide from LAP protein
<400> 2
   Gln Arg Val Ile Tyr Tyr Tyr Ala Ala Ala Gln Thr Thr His Thr Thr
   1 5 10 15
   Tyr Pro Glu Gly Leu Glu Val Leu His Phe Ser Ser Gly Gln Ile Glu
   20 25 30
   Lys His Tyr Pro Asp Gly Arg Lys Glu Ile Thr Phe Pro Asp Gln Thr
   35 40 45
   Val Lys Asn Leu Phe Pro Asp Gly Gln Glu Glu Ser Ile Phe Pro Asp
   50 55 60
   Gly Thr Ile Val Arg Val Gln Arg Asp Gly Asn Lys Leu Ile Glu Phe
   65 70 75 80
   Asn Asn Gly Gln Arg Glu Leu His Thr Ala Gln Phe Lys Arg Arg Glu
   85 90 95
   Tyr Pro Asp Gly Thr Val Lys Thr Val Tyr Ala Asn Gly His Gln Glu
   100 105 110
   Thr Lys Tyr Arg Ser Gly Arg Ile Arg Val Lys Asp Lys Glu Gly Asn
   115 120 125
   Val Leu Met Asp Thr Glu Leu
   130 135
<210> 3
   <211> 18
   <212> PRT
   <213> Artificial sequence
<220>
   <221> misc-feature
   <222> (1)..(18)
   <223> repeated EP motif
<220>
   <221> misc-feature
   <222> (1)..(18)
   <223> corresponds to EP motifs from human LAG-3 protein.
<400> 3
   Glu Pro Glu Pro Glu Pro Glu Pro Glu Pro Glu Pro Glu Pro Glu Pro
   1 5 10 15
   Glu Pro
<210> 4
   <211> 12
   <212> PRT
   <213> artificial sequence
<220>
   <221> misc-feature
   <222> (1)..(12)
   <223> repeated EP motif
<220>
   <221> misc-feature
   <222> (1)..(12)
   <223> correpsponds to EP motifs from mLAG-3 protein.
<400> 4
   Glu Pro Glu Pro Glu Pro Gln Leu Glu Pro Glu Pro
   1               5                   10
<210> 5
   <211> 23
   <212> PRT
   <213> artificial sequence
<220>
   <221> misc-feature
   <222> (1) .. (23)
   <223> repeated EP motif
<220>
   <221> misc-feature
   <222> (1)..(23)
   <223> corresponds to EP motifs from PDGFR protein.
<400> 5
   Glu Pro Gln Asp Glu Pro Pro Glu Pro Gln Leu Glu Leu Gln Val Glu
   1               5                   10                  15
   Pro Glu Pro Glu Leu Glu Gln
               20
<210> 6
   <211> 12
   <212> PRT
   <213> artificial sequence
<220>
   <221> misc-feature
   <222> (1)..(12)
   <223> repeated EP motif
<220>
   <221> misc-feature
   <222> (1)..(12)
   <223> Corresponds to EP motifs from HS1 protein.
<400> 6
   Glu Pro Glu Pro Glu Pro Glu Pro Glu Pro Glu Pro
   1               5                   10
<210> 7
   <211> 486
   <212> PRT
   <213> homo sapiens
<300>
   <301> Kitamura, D. et al.
   <302> Isolation and characterization of a novel human gene expressed specifically in the cells of hematopietic lineage.
   <303> Nucleic Acid research
   <304> 17
   <305> 22
   <306> 9367-9379
   <307> 1989-_-_
   <308> s07633
   <309> 1993-01-29
   <313> (1) .. (486)
<400> 7
<210> 8
   <211> 1353
   <212> DNA
   <213> homo sapiens
<220>
   <221> misc feature
   <222> (1) .. (1353)
   <223> LAP cDNA Open reading frame.
<220>
   <221> misc_feature
   <222> (1186)..(1189)
   <223> LAP cDNA translation codon stop
<400> 8
   gaaattgcag acttcgaaca acagaaagca aaagaattag ctcgaataga agagtttaaa     60
   aaggaggaga tgaggaagct acaaaaggaa cgtaaagttt ttgaaaagta tactacagct    120
   gcaagaactt ttccagataa aaaggaacgt gaagaaatac agactttaaa acagcaaata    180
   gcagatttac gggaagattt gaaaagaaag gaaaccaaat ggtcaagtac acacagccgt    240
   ctcagaagcc agatacaaat gttagtcaga gagaacacag acctccggga agaaataaaa    300
   gtgatggaaa gattccgact ggatgcctgg aagagagcag aagccataga gagcagcctc    360
   gaggtggaga agaaggacaa gcttgcgaac acatctgttc gatttcaaaa cagtcagatt    420
   tcttcaggaa cccaggtaga aaaatacaag aaaaattatc ttccaatgca aggcaatcca    480
   cctcgaagat ccaagtctgc acctcctcgt gatttaggca atttggataa gggacaagct    540
   gcctctccca gggagccact tgaaccactg aacttcccag atcctgaata taaagaggag    600
   gaggaagacc aagacataca gggagaaatc agtcatcctg atggaaaggt ggaaaaggtt    660
   tataagaatg ggtgccgtgt tatactgttt cccaatggaa ctcgaaagga agtgagtgca    720
   gatgggaaga ccatcactgt cactttcttt aatggtgacg tgaagcaggt catgccagac    780
   caaagagtga tctactacta tgcagctgcc cagaccactc acacgacata cccggaggga    840
   ctggaagtct tacatttctc aagtggacaa atagaaaaac attacccaga tggaagaaaa    900
   gaaatcacgt ttcctgacca gactgttaaa aacttatttc ctgatggaca agaagaaagc    960
   attttcccag atggtacaat tgtcagagta caacgtgatg gcaacaaact catagagttt   1020
   aataatggcc aaagagaact acatactgcc cagttcaaga gacgggaata cccagatggc   1080
   actgttaaaa ccgtatatgc aaacggtcat caagaaacga agtacagatc cggtcggata   1140
   agagttaagg acaaggaggg taatgtgcta atggacacgg agctgtgacg atcctcatgt   1200
   gatcatgaag taacagtaac tgacttttta tgttaaaaaa tgtacattta ctgtggattc   1260
   tgtttaattt attgtgtatg tgtggggaaa agattggatt ctaaaataaa agtttaccct   1320
   gtggcatctt catttttata ttctttgaaa tgc                                 1353
<210> 9
   <211> 17
   <212> PRT
   <213> artificial sequence
<220>
   <221> misc-feature
   <222> (1) .. (17)
   <223> LAP derived peptide. Contains LAP epitope to raise specific LAP antibodies
<400> 9
   Ser Pro Arg Glu Pro Leu Glu Pro Leu Asn Phe Pro Asp Pro Glu Tyr
   1               5                   10                  15
   Lys